# EUROPÄISCHE PATENTANMELDUNG

(11) **EP 2 014 314 A1**
(43) Veröffentlichungstag der Anmeldung: **14.01.2009**
(21) Anmeldenummer: 07013425.9
(22) Anmeldetag: 10.07.2007
(51) Int. Cl.: A61L 15/42, C08L 75/00

(54) **Verfahren zur Herstellung von Polyurethan-Schäumen für die Wundbehandlung**

(71) Anmelder: Bayer Innovation GmbH, 40225 Düsseldorf (DE)
(72) Erfinder: Mager, Michael, 51375 Leverkusen (DE); Ludewig, Michael, 51373 Leverkusen (DE); Matner, Mathias, 41464 Neuss (DE); Dietze, Melita, 40699 Erkrath (DE); Fugmann, Burkhard, 40878 Ratingen (DE)

(57) **Zusammenfassung**

Die vorliegende Erfindung betrifft neuartige Wundauflagen auf Polyurethan-Basis, welche durch Aufschäumen und Aushärten von silanterminierten Polyurethanen erhalten werden.

## Beschreibung

Die vorliegende Erfindung betrifft neuartige Wundauflagen auf Polyurethan-Basis, welche durch Aufschäumen und Aushärten von silanterminierten Polyurethanen erhalten werden.

Die Verwendung von Wundauflagen aus Schäumen zur Behandlung von nässenden Wunden ist Stand der Technik. Aufgrund ihres hohen Absorptionsvermögens und ihrer guten mechanischen Eigenschaften werden in Regel Polyurethan-Schäume eingesetzt, welche durch Umsetzung von Mischungen aus Diisocyanaten und Polyolen bzw. NCO-funktionellen Polyurethan-Prepolymeren mit Wasser in Gegenwart bestimmter Katalysatoren sowie (Schaum-)Additiven hergestellt werden. In der Regel werden hierbei aromatische Diisocyanate eingesetzt, da sich diese am besten Verschäumen lassen. Zahlreiche Ausführungsformen dieser Verfahren sind bekannt, beispielsweise beschrieben in US 3,978,266, US 3,975,567 und EP-A 0 059 048. Die vorgenannten Verfahren weisen jedoch den Nachteil auf, dass reaktive Mischungen eingesetzt werden müssen, enthaltend Diisocyanate oder entsprechende NCO-funktionelle Prepolymere, deren Handhabung technisch aufwendig ist, da z.B. entsprechende Schutzmaßnahmen erforderlich sind. Ein direkter Auftrag dieser Mischungen auf die (menschliche) Haut ist aufgrund der hohen Reaktivität der vorhandenen Isocyanatgruppen nicht möglich.

Neben der Verwendung von Zusammensetzungen, welche freie Isocyanatgruppen enthalten, können Polyurethan-Schäume auch unter Einsatz von silanterminierten Polyurethan-Prepolymeren hergestellt werden, welche sich durch Verwendung von Treibmitteln aufschäumen lassen. Zur Herstellung von Dicht- und Dämmschäumen sind zahlreiche Ausführungsformen bekannt, beispielsweise beschrieben in der EP-A 1 098 920. Die Verwendung der Zusammensetzungen zur Herstellung von Wundauflagen wurde bislang nicht erkannt. Für die Eignung als Wundauflagen kritische Anforderungen wie gute Wasserdampfpermeabilität und hohes Flüssigkeitsaufnahmevermögen wurden ebenfalls bisher nicht beschrieben.

Aufgabe der vorliegenden Erfindung war es nun Wundauflagen aus Polyurethanen bereitzustellen, welche die eingangs erwähnten Nachteile des Einsatzes Isocyanatgruppen-haltiger, reaktiver Mischungen vermeiden.

Es wurde nun überraschenderweise gefunden, dass sich silanterminierte Polyurethan-Prepolymere (STP's) nach Aufschäumen und Aushärtung durch Vernetzung der Silangruppen ebenfalls hervorragend als Wundauflagen für medizinische Anwendungen einsetzen lassen.

Gegenstand der vorliegenden Erfindung ist daher die Verwendung von Schäumen erhältlich aus silanterminierten Polyurethan-Prepolymeren als Wundauflagen.

Ein weiterer Gegenstand der Erfindung ist ein Verfahren zur Herstellung von Wundauflagen, bei dem eine Zusammensetzung enthaltend
a) silanterminierte Polyurethan-Prepolymere (I)
b) (Schaum-)Additive (II)
c) gegebenenfalls Katalysatoren (III),
d) gegebenenfalls Treibmittel (IV) sowie
e) gegebenenfalls weitere Hilfs- und Zusatzstoffe (V)
aufschäumt, vor, während oder nach dem Aufschäumen auf ein Substrat aufgebracht und schließlich in Anwesenheit von Wasser ausgehärtet wird.

Ferner sind ein Gegenstand der vorliegenden Erfindung die nach dem erfindungsgemäßen Verfahren erhältlichen Wundauflagen.

Darüber hinaus ist ein Gegenstand der Erfindung die Verwendung von Zusammensetzungen enthaltend
a) silanterminierte Polyurethan-Prepolymere (I) umfassend mehr als eine Alkoxysilangruppe
b) (Schaum-)Additive (II)
c) gegebenenfalls Katalysatoren (III),
d) gegebenenfalls Treibmittel (IV) sowie
e) gegebenenfalls weitere Hilfs- und Zusatzstoffe (V)
für die Herstellung von Auflagen für die Wundbehandlung.

Wundauflagen aus Polyurethan-Schäumen im Sinne der Erfindung sind poröse Materialien, bevorzugt mit zumindest teilweise vorhandener Offenzelligkeit, welche im wesentlichen aus über Siloxanbindungen Si-O-Si vernetzen Polyurethanen bestehen und Wunden im Sinne einer Abdeckung vor Keimen bzw. Umgebungseinflüssen schützen, eine schnelle und hohe Absorption von physiologischer Salzlösung bzw. Wundflüssigkeit aufweisen und durch geeignete Feuchtedurchlässigkeit für ein optimales Wundklima sorgen.

Silanterminierte Polyurethan-Prepolymere (I) im Sinne der Erfindung sind alle (Pre-)Polymere mit mehr als einer Urethan- und mehr als einer Alkoxysilangruppe, welche in Gegenwart von Wasser und gegebenenfalls Katalysatoren (III) über Siloxanbindungen Si-O-Si zu vernetzten Polyurethanen reagieren können.

Solche silanterminierten Prepolymere sind erhältlich, indem
i) freie Isocyanatgruppen aufweisende Polyurethan-Prepolymere (A) mit einer mittleren NCO-Funktionalität von mindestens 1,5 mit
ii) Amino-, Hydroxy- und/oder Thiolgruppen aufweisenden Di- und/oder Trilkoxysilanen (B), wobei die Amino-, Hydroxy- und/oder Thiolgruppen über einen Alkylenrest an das Siliciumatom gebunden sind
   oder
iii) Polyhydroxyverbindungen (C) mit einer mittleren OH-Funktionalität von mindestens 1,5 mit
iv) Isocyanat- und/oder Isothiocyanatgruppen aufweisenden Di- und/oder Trialkoxysilanen (D), wobei die Isocyanat- und/oder Isothiocyanatgruppen über einen Alkylenrest an das Siliciumatom gebunden sind
umgesetzt werden.

Die freie Isocyanatgruppen aufweisenden Polyurethan-Prepolymere (A) sind in an sich bekannter Weise durch die Umsetzung organischer Polyisocyanate mit Polyhydroxyverbindungen einer Funktionalität von 1,5 bis 6 erhältlich, wobei die organischen Polyisocyanate im Überschuss eingesetzt werden (Molverhältnis NCO/OH > 1).

Geeignete Polyisocyanate sind die dem Fachmann an sich bekannten aromatischen, araliphatischen, aliphatischen oder cycloaliphatischen Polyisocyanate einer mittleren NCO-Funktionalität von ≥ 2.

Beispiele sind 1,4-Butylendiisocyanat, 1,6-Hexamethylendiisocyanat (HDI), Isophorondiisocyanat (IPDI), 2,2,4 und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3-und/oder 1,4-Bis-(2-isocyanato-prop-2-yl)-benzol (TMXDI), 1,3-Bis(isocyanatomethyl)benzol (XDI), Alkyl-2,6-diisocyanatohexanoat (Lysindiisocyanate) mit C1-C8-Alkylgruppen, sowie 4-Isocyanatomethyl-1,8-octandiisocyanat (Nonantriisocyanat) und Triphenylmethan-4,4',4"-triisocyanat.

Neben den vorstehend genannten Polyisocyanaten können anteilig auch modifizierte Diisocyanate oder Triisocyanate mit Uretdion-, Isocyanurat-, Urethan-, Allophanat-, Biuret-, Iminooxadiazindion- und/oder Oxadiazintrionstruktur mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität von 2 bis 4, bevorzugt 2 bis 2,6 und besonders bevorzugt 2 bis 2,4. Besonders bevorzugt werden 1,6-Hexamethylendiisocyanat, Isophorondiisocyanat, die isomeren Bis(4,4'-isocyanatocyclohexyl)methane, sowie deren Mischungen eingesetzt.

Als Polyhydroxyverbindungen der Komponente (C) können alle an sich in der Polyurethanlacktechnologie bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole eingesetzt werden, die eine mittlere OH-Funktionalität von mindestens 1,5 aufweisen. Diese können einzeln oder in beliebigen Mischungen untereinander eingesetzt werden. Bevorzugt werden jedoch Polyetherpolyole eingesetzt.

Polyesterpolyole der Komponente (C) sind die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Zur Herstellung der Polyesterpolyole geeignete Diole sind beispielsweise Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopenthylglykolester bevorzugt sind. Als Tri- und Tetraaole sind beispielsweise geeignet Trimethylolpropan, Glycerin, Erythrit, Pentaerythrit, Triemthylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbemsteinsäure, 3,3-Diethylglutarsäure und/oder 2,2-Dimethylbernsteinsäure eingesetzt werden. Als Säurequelle können auch die entsprechenden Anhydride verwendet werden. Bevorzugte Säuren sind aliphatische oder aromatische Säuren der vorstehend genannten Art. Besonders bevorzugt sind Adipinsäure, Isophthalsäure und gegebenenfalls Trimellithsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Als Polyhydroxyverbindungen der Komponente (C) können weiterhin Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind in an sich bekannter Weise durch Reaktion von Kohlensäurederivaten, wie Diphenylcarbonat, Dimethylcarbonat oder Phosgen, mit Polyolen, bevorzugt Diolen, erhältlich.

Zur Herstellung der Polycarbonate geeignete Diole sind beispielsweise Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält das Polycarbonatdiol 40 bis 100 Gew.-% Hexandiol bevorzugt 1,6-Hexandiol und/oder Hexandiolderivate, bezogen auf die zugrunde liegenden Diole. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Als Polyhydroxyverbindungen der Komponente (C) können überdies Polyetherpolyole eingesetzt werden. Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Bevorzugte Polyetherpolyole der Komponente (C) sind die an sich bekannten Additionsprodukte von Styroloxid, Ethylenoxid, Propylenoxid, Butylenoxide und/oder Epichlorhydrin an di- oder polyfunktionelle Startermoleküle, wobei Additionsprodukte aus Ethylenoxid oder Propylenoxid, sowie Gemische der genannten, besonders bevorzugt sind. Ganz besonders bevorzugt sind Additionsprodukte aus Ethylenoxid und Propylenoxid, in welchen der Gewichtsanteil von Ethylenoxid mindestens 5 bis 8 Gew.- %, bevorzugt 10 bis 65 Gew.%, besonders bevorzugt 10 bis 30 Gew.-% beträgt, wobei dieser Gewichtsanteil bezogen ist auf die insgesamt im Additionsprodukt vorliegenden Ethylenoxid- und Propylenoxideinheiten sowie die eingesetzten Startermoleküle.

Die Alkoxylierung mit Ethylenoxid bzw. Propylenoxid kann dabei unter Basenkatalyse oder unter Einsatz von Doppelmetallcyanidverbindungen (DMC-Verbindungen) erfolgen.

Als geeignete Startermoleküle zur Herstellung der Polyetherpolyole der Komponente (C) können alle dem Stand der Technik nach bekannten niedermolekulare Polyole, organische Polyamine und/oder Wasser eingesetzt werden, wie zum Beispiel Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol, Butyldiglykol oder beliebige Gemische der vorgenannten.

Bevorzugt weisen die Polyetherpolyole zahlenmittlere Molekulargewichte Mₙ von 300 bis 20 000 g/mol, besonders bevorzugt 1 000 bis 12 000, ganz besonders bevorzugt 2 000 bis 6 000 g/mol auf.

Durch Umsetzung organischer Di- oder Polyisocyanate mit Polyhydroxyverbindungen einer Funktionalität 1,5 bis 6 im Molverhältnis NCO/OH < 1 erhält man Urethangruppen aufweisende Polyhydroxyverbindungen, die ebenfalls als Komponente (C) eingesetzt werden können.

Geeignete Amino-, Hydroxy- und/oder Thiolgruppen aufweisende Di- und/oder Trilkoxysilane der Komponente (B) sind dem Fachmann hinlänglich bekannt, beispielhaft genannt seien Aminopropyltrimethoxysilan, Mercaptopropyltrimethoxysilan, Aminopropylmethyldimethoxysilan, Mercaptopropylmethyldimethoxysilan, Aminopropyltriethoxysilan, Mercaptopropyltriethoxysilan, Aminopropylmethyldiethoxysilan, Mercaptopropylmethyldiethoxysilan, Aminomethyltrimethoxysilan, Aminomethyltriethoxysilan, (Aminomethyl)methyldimethoxysilan, (Aminomethyl)methyl-diethoxysilan, *N*-Butyl-aminopropyltrimethoxysilan, *N*-Ethyl-aminopropyltrimethoxysilan, *N-*Phenyl-aminopropyltrimethoxysilan, *N*-(3-Triethoxysilylpropyl)asparaginsäurediethylester, *N*-(3-Trimethoxysilylpropyl)asparaginsäurediethylester und *N*-(3-Dimethoxymethylsilylpropyl)aspara-ginsäurediethylester. Bevorzugt ist die Verwendung von *N*-(3-Trimethoxysilylpropyl)-asparaginsäurediethylester und Aminopropyltrimethoxysilan.

Geeignete Isocyanat- und/oder Isothiocyanatgruppen aufweisende Di- und/oder Trialkoxysilane der Komponente (D) sind ebenfalls grundsätzlich bekannt.

Als Beispiele genannt seien Isocyanatomethyltrimethoxysilan, Isocyanatomethyltriethoxysilan, (Isocyanatomethyl)methyldimethoxysilan, (Isocyanatomethyl)methyldiethoxysilan, 3-Isocyanatopropyltrimethoxysilan, 3-Isocyanatopropylmethyldimethoxysilan, 3-Isocyanatopropyltriethoxysilan und 3-Isoeyanatopropylmethyldiethoxysilan. Bevorzugt ist hier die Verwendung von 3-Isocyanatopropyltrimethoxysilan und 3-Isocyanatpropyltriethoxysilan.

Die Additive (II) sind nichtionische, anionische, kationische oder zwitterionische Tenside oder Mischungen der genannten Tenside, welche in den erfindungsgemäßen Zusammensetzungen der Verbesserung der Schaumbildung, der Schaumstabilität oder der Eigenschaften des resultierenden Polyurethan-Schaums dienen. Bevorzugte Additive (II) sind nichtionische, besonders bevorzugt nichtionische Tenside auf Basis von Polyethersiloxanen.

Die bevorzugten Additive (II) besitzen neben der stabilisierenden Wirkung auch einen besonders vorteilhaften Einfluss in Bezug auf die Hydrophilierung der Schäume was sich hinsichtlich Menge und Geschwindigkeit der Feuchtigkeitsaufnahme bemerkbar macht.

Als Katalysatoren (III) können den erfindungsgemäßen Zusammensetzungen grundsätzlich alle aus der Siliziumchemie an sich bekannten Stoffe zugesetzt werden, welche die Hydrolyse und Kondensation von Alkoxysilanen bzw. Silanolgruppen katalysieren. Genannt seien beispielsweise Metallsalze, Metallkomplexe, metallorganische Verbindungen, sowie Säuren und Basen. Bevorzugt ist die Verwendung von organischen und anorganischen Säuren oder Basen, besonders bevorzugt die Verwendung organischer oder anorganischer Säuren wie beispielsweise Salzsäure oder p-Toluolsulfonsäure. Sofern Katalysatoren (III) in den Zusammensetzungen verwendet werden, so werden sie bevorzugt in Wasser gelöst, welches gleichzeitig für die eigentliche Vernetzung der Schäume notwendig ist.

Als Treibmittel (IV) kann im einfachsten Falle Luft oder Stickstoff eingesetzt werden, jedoch können selbstverständlich auch alle anderen, aus der Polyurethanchemie an sich bekannten, Treibmittel zum Aufschäumen der erfindungsgemäßen Zusammensetzung eingesetzt werden. Genannt seien beispielsweise n-Butan, i-Butan, Propan und Dimethylether, sowie Mischungen der vorgenannten.

Als Hilfs- und Zusatzstoffe (V) können beispielsweise Füllstoffe, Verdicker bzw. Thixotropiermittel, Antioxidantien, Lichtschutzmittel, Weichmacher, Pigmente, und/oder Verlaufshilfsmittel eingesetzt werden.

Bevorzugte Hilfs- und Zusatzstoffe sind Füllstoffe, bevorzugt anorganische Füllstoffe, welche zu einer Verbesserung der mechanischen Eigenschaften des erfindungsgemäßen Polyurethan-Schaums beitragen können. Geeignet sind beispielsweise Kreiden und hochdisperse Kieselsäuren, insbesondere flammpyrolytisch hergestellte Kieselsäuren.

Als Weichmacher könne alle natürlichen oder synthetischen Stoffe eingesetzt werden, welche mit dem Polyurethan-Schaum eine genügend gute Verträglichkeit zeigen. Beispiele geeigneter Weichmacher sind Campher, Ester (aliphatischer) Dicarbonsäuren, z.B. der Adipinsäure, Polyester, insbesondere auf Basis von Adipin-, Sebacin-, Azelain und Phtalsäuresäure kondensiert mit 1,3-Butandiol, 1,4-Butandiol oder 1,6-Hexandiol, sowie Phosphorsäureester, Fettsäureester und Hydroxycarbonsäureester (z.B. auf Basis von Zitronensäure, Weinsäure oder Milchsäure).

Die Aushärtung unter Vernetzung der Alkoxysilangruppen zu Siloxanbrücken erfolgt unter Einwirkung von Wasser, welches in flüssiger Form, z.B. als Lösemittel für den Katalysator, direkt zugesetzt werden kann oder aus der Luft in Form von Luftfeuchtigkeit kommen kann.

Die erfindungswesentlichen Zusammensetzungen enthalten, bezogen auf Trockensubstanz, typischerweise 80 bis 99,9 Gewichtsteile des silanterminierten Polyurethan-Prepolymers (I) und 0,1 bis 20 Gewichtsteile des (Schaum-) Additivs (II). Bevorzugt enthalten die Zusammensetzungen, bezogen auf Trockensubstanz, 85 bis 99,9 Gewichtsteile silanterminierten Polyurethan-Prepolymers (I) und 0,1 bis 15 Gewichtsteile des (Schaum-) Additivs (II), besonders bevorzugt 95 bis 99,9 Gewichtsteile (I) und 0,1 bis 5 Gewichtsteile (II).

Hilfs- und Zusatzstoffe (V) werden typischerweise in Mengen von 0 bis 50 Gewichtsteilen zugegeben, bevorzugt 10 bis 40 Gewichtsteilen.

Wird für die Vernetzung über die Umgebungsfeuchtigkeit hinaus Wasser zugesetzt, so wird es typischerweise in einer Menge zugegeben, dass das Molverhältnis von Alkoxygruppen zu zugesetztem Wasser kleiner oder gleich 1 ist (Überschuss Wasser). Bevorzugt ist das Molverhältnis kleiner oder gleich 0,75, besonders bevorzugt kleiner oder gleich 0,55.

Das Treibmittel oder das Treibmittelgemisch (IV) wird typischerweise in einer Menge von 1 bis 50 Gew.-%, bevorzugt 5 bis 40 Gew.-% und besonders bevorzugt 5 bis 20 Gew.-% eingesetzt, wobei die Summe der eingesetzten Komponenten (1), (II) und gegebenenfalls (III), (IV), (V), 100 Gew.-% ergibt.

Das Mischen der Komponenten (I) und (II) kann in beliebiger Reihenfolge erfolgen, ebenso wie die Mischung mit den gegebenenfalls enthaltenen Komponenten (III) bis (V).

Bevorzugt werden die Komponenten (I) und (II) sowie gegebenenfalls (III) bis (V) jeweils getrennt bereitgestellt, das heisst die Reaktivkomponente (I) wird in Abwesenheit von Wasser und dem gegebenenfalls einzusetzenden Katalysator (III) bereitgestellt. Damit kann eine mindestens 6 Monate bei 23 °C lagerstabile Zusammensetzung erhalten werden (Als lagerstabil wird ein Viskositätsanstieg der Mischung von weniger als 50 %, bevorzugt weniger als 25 % und besonders bevorzugt weniger als 15 % bezogen auf das Ausgangsniveau der Zusammensetzung verstanden).

Das Aufschäumen im erfindungsgemäßen Verfahren geschieht durch Schütteln der Zusammensetzung, mechanisches Rühren bei hohen Drehzahlen oder durch Entspannung eines Treibgases. Nach oder während des Aufschäumens, erfolgt die Aushärtung der Zusammensetzung, wonach der gewünschte Polyurethan-Schaum erhalten wird. Vor der vollständigen Erstarrung bzw. Aushärtung, d.h. solange die Zusammensetzung noch fließfähig ist, kann diese durch gängige Applikationstechniken wie Gießen oder Rakeln auf ein geeignetes Substrat aufgebracht werden. Überdies kann auch ein direkter Auftrag der Zusammensetzung auf die menschliche oder tierische Haut erfolgen, wobei das Aufschäumen und die Aushärtung in der Regel dann gleichzeitig erfolgt.

Das mechanische Aufschäumen kann mit beliebigen mechanischen Rühr-, Misch- und Dispergiertechniken erfolgen. In der Regel wird hierbei Luft eingetragen, aber auch Stickstoff und andere Gase können hierfür benutzt werden.

Der so erhaltene Schaum wird beim Aufschäumen oder unmittelbar danach auf ein Substrat aufgetragen oder in eine Form gegeben und ausgehärtet.

Der Auftrag kann beispielsweise durch Gießen oder Rakeln erfolgen, aber auch andere an sich bekannte Techniken sind möglich. Ein mehrschichtiger Auftrag mit gegebenenfalls zwischengeschalteten Aushärtungsschritten ist grundsätzlich auch möglich.

Eine befriedigende Aushärtungsgeschwindigkeit der Schäume wird bereits bei 20°C beobachtet. Für eine schnellere Aushärtung und Fixierung der Schäume können jedoch auch höhere Temperaturen von bevorzugt mehr als 30 °C angewandt werden, z.B. unter Zuhilfenahme von an sich bekannten Heiz- und Trockenapparaten, wie (Umluft-) Trockenschränken, Heißluft oder IR-Strahlern.

Die Zusammensetzungen können bereits aufgeschäumt oder unter Aufschäumen unmittelbar auf die Haut oder im Rahmen einer industriellen Herstellung von Wundauflagen auf Trennpapiere oder Folien aufgebracht werden, die ein einfaches Ablösen der Wundauflage vor ihrem Einsatz zur Abdeckung einer verletzten Stelle ermöglichen.

Der Auftrag sowie die Aushärtung können jeweils diskontinuierlich oder kontinuierlich durchgeführt werden, im Fall der industriellen Herstellung von Wundauflagen ist jedoch ein gänzlich kontinuierliches Verfahren bevorzugt.

Bei einem direkten Auftrag der Zusammensetzung, z.B. durch Sprühen, auf die tierische oder menschliche Haut, erfolgt die Aushärtung ebenfalls bereits sehr rasch bei Umgebungsbedingungen bzw. durch die Körpertemperatur. Die Zuhilfenahme externer Wärmequelle ist hier ebenso möglich, wenn auch nicht bevorzugt.

In einer Ausführungsform der vorliegenden Erfindung wird das silanterminierte Polyurethan-Prepolymer (I) mit dem Additiv (II) und gegebenenfalls weiteren Hilfs- und Zusatzstoffen (V) gemischt. Nach dem Aufschäumen der Mischung, was beispielsweise durch mechanischen Eintrag von Luft oder eines anderen Gases erfolgen kann, wird der Katalysator (III) zugegeben, die (geschäumte) Mischung auf ein geeignetes Substrat aufgetragen und schließlich in Gegenwart von Luftfeuchte ausgehärtet. Um die Aushärtung der geschäumten Mischung zu beschleunigen, kann überdies Wasser zugegeben werden, was vorzugsweise zusammen mit dem (gelösten) Katalysator (III) erfolgt.

In einer weiteren Ausführungsform der vorliegenden Erfindung wird das silanterminierte Polyurethan-Prepolymer (I) mit dem Additiv (II) und gegebenenfalls weiteren Hilfs- und Zusatzstoffen (V) gemischt und in einen geeigneten Druckbehälter, z.B. eine Sprühdose, überführt. Danach wird das Treibmittel (IV) zugegeben; während der Applikation der Mischung auf ein geeignetes Substrat erfolgt das Aufschäumen und die Aushärtung durch Luftfeuchte.

In einer weiteren Ausführungsform der vorliegenden Erfindung wird das silanterminierte Polyurethan-Prepolymer (I) mit dem Additiv (II) und gegebenenfalls weiteren Hilfs- und Zusatzstoffen (V) gemischt und in eine erste Kammer eines geeigneten Druckbehälters, z.B. eine Sprühdose, überführt, wobei der Drückbehälter über mindestens 2 getrennte Kammern verfügt. In eine zweite Kammer des Druckbehälters wird der Katalysator (III) gegeben, welcher bevorzugt mit einer geeigneten Menge Wasser vermischt ist. Die Hilfs- und Zusatzstoffe (V) können auch in der zweiten Kammer zugemischt werden, dies ist jedoch weniger bevorzugt. Nun wird das Treibmittel (IV) zu einer oder beiden der Kammern zugegeben und schließlich erfolgt die Applikation der Zweikomponentenmischung auf ein geeignetes Substrat, wobei gleichzeitig das Aufschäumen sowie die Aushärtung erfolgt.

Die Polyurethan-Schäume haben vor ihrer Aushärtung typischerweise Schaumdichten von 50 bis 800 g/Liter, bevorzugt 100 bis 500 g/Liter, besonders bevorzugt 100 bis 250 g/Liter (Masse aller Einsatzstoffe [in g] bezogen auf das Schaumvolumen von einem Liter).

Die Polyurethan-Schäume besitzen nach ihrer Trocknung eine mikroporöse, zumindest teilweise offenporige Struktur mit miteinander kommunizierenden Zellen. Die Dichte der Ausgehärteten Schäume liegt dabei typischerweise unterhalb von 0,4 g/cm³, bevorzugt ist sie geringer als 0,35 g/cm³ und liegt besonders bevorzugt bei 0,01 bis 0,2 g/cm³.

Das Absorptionsvermögen gegenüber physiologischer Salzlösung beträgt bei den Polyurethan-Schäumen typischerweise 100 und 1500 %, bevorzugt 300 bis 800 % (Masse der aufgenommen Flüssigkeit bezogen auf die Masse des trockenen Schaums; Bestimmung nach DIN EN 13726-1,. Teil 3.2). Die Durchlässigkeit gegenüber Wasserdampf beträgt typischerweise 500 bis 8000 g/24 h * m², bevorzugt 1000 bis 6000 g/24 h * m² und besonders bevorzugt 2000 bis 5000 g/24 h * m² (Bestimmung nach DIN EN 13726-2, Teil 3.2).

Die Polyurethan-Schäume weisen eine gute mechanische Festigkeit und hohe Elastizität auf. Typischerweise sind die Werte für die maximale Spannung größer als 0,1 N/mm² und die maximale Dehnung ist größer als 100 %. Bevorzugt ist die die Dehnung größer als 200 % (Bestimmung nach DIN 53504).

Die Polyurethan-Schäume haben nach der Aushärtung typischerweise eine Dicke von 0,1 mm bis 50 mm, bevorzugt 0,5 mm bis 20 mm, besonders bevorzugt 1 bis 10 mm, ganz besonders bevorzugt 1 bis 5 mm.

Die Polyurethan-Schäume können überdies mit weiteren Materialien verklebt, laminiert oder beschichtet werden, beispielsweise auf Basis von Hydrogelen, (semi-) permeablen Folien, Beschichtungen, Hydrokolloiden oder anderen Schäumen.

Ebenfalls möglich ist die Zugabe, Einarbeitung oder Beschichtung von bzw. mit antimikrobiellen oder biologischen Wirkstoffen, welche sich beispielsweise in Bezug auf die Wundheilung und die Vermeidung von Keimbelastungen positiv auswirken.

### Beispiele:

Sofern nicht abweichend gekennzeichnet, beziehen sich alle Prozentangaben auf das Gewicht.

NCO-Gehalte wurden, wenn nicht ausdrücklich anders erwähnt, volumetrisch gemäß DIN-EN ISO 11909 bestimmt.

### Verwendete Abkürzungen und Einsatzstoffe:

- PO: Propylenoxid
- EO: Ethylenoxid
- DBTL: Dibutylzinndilaurat
- Tegostab^{®} B 1048: Polyethersiloxan (Fa. Degussa, Düsseldorf, DE)
- Aerosil^{®} R 9200: Flammpyrolytisch hergestellte, hochdisperse Kieselsäure (Fa. Degussa, Düsseldorf, DE)
- Mesamoll^{®}: Weichmacher auf Basis eines Alkylsulfonsäureesters (Fa. Lanxess, Leverkusen, DE)

### Herstellung des silanterminierten Prepolymers 1 (STP 1):

Eine Mischung aus 2003,6 g eines difunktionellen, ethylenoxidhaltigen Polyethers (OH-Zahl 28, Molgewicht 4000 g/mol, Verhältnis PO/EO=6,5), 214,3 g 3-Isocyanatotrimethoxysilan und 133 µl DBTL wurde solange unter Rühren auf 60 °C erwärmt bis der NCO-Gehalt bei 0 lag.

### Beispiel 1: Herstellung eines Schaums unter Basenkatalyse

117,5 g des STP's 1 und 3,8 g Tegostab^{®} B 1048 wurden in einem Kunststoffbecherglas mit einem Handrührer vermischt und in 10 Min. auf eine Volumen von ca. 300 ml aufgeschäumt. Danach wurden 2,5 g Kalilauge (1,25 mol/L) zugegeben, wonach innerhalb von 20 s die Aushärtung erfolgte. Es wurde ein weißer Schaum erhalten.

### Beispiel 2: Herstellung von Schäumen unter Säurekatalyse

a) 117,5 g des STP's 1 und 3,8 g Tegostab^{®} B 1048 wurden in einem Kunststoffbecherglas mit einem Handrührer vermischt und in 10 Min. auf eine Volumen von ca. 300 ml aufgeschäumt. Danach wurden 2,5 g einer 5 %igen wässrigen Lösung von p-Toluolsulfonsäure zugegeben, wonach innerhalb von 100 s die Aushärtung erfolgte. Es wurde ein weißer Schaum erhalten.
b) Der Versuch wie in a) beschrieben wurde mit 2 g einer 20 %igen wässrigen Lösung von p-Toluolsulfonsäure durchgeführt, wobei die Aushärtung zu einem weißen Schaum bereits nach 50 s erfolgte.

### Beispiel 3: Schaum mit Füllstoff und Weichmacher

Mittels eines Dissolvers wurden zunächst 50 g Aerosil^{®} R 9200 in 117,5 g des STP's 1 dispergiert (nahezu transparente Dispersion). Danach wurden 25 g Mesamoll^{®} und 3,8 g Tegostab^{®} B 1048 zugegeben, schließlich wurde die Mischung in einem Kunststoffbecherglas mit einem Handrührer in 10 Min. auf eine Volumen von ca. 300 ml aufgeschäumt. Nach Zugabe von 2,5 g einer 5 %igen wässrigen Lösung von p-Toluolsulfonsäure, wurde innerhalb von 20 s die Aushärtung zu einem weißen Schaum erreicht.

## Patentansprüche

1. Verwendung von Schäumen erhältlich aus silanterminierten Polyurethan-Prepolymeren als Wundauflagen.

2. Verfahren zur Herstellung von Wundauflagen, bei dem in dem eine Zusammensetzung enthaltend
a) silanterminierte Polyurethan-Prepolymere (I) umfassend mehr als eine Alkoxysilangruppe
b) (Schaum-)Additive (II)
c) gegebenenfalls Katalysatoren (III),
d) gegebenenfalls Treibmittel (IV) sowie
e) gegebenenfalls weitere Hilfs- und Zusatzstoffe (V)
aufschäumt, vor, während oder nach dem Aufschäumen auf ein Substrat aufgebracht und schließlich in Anwesenheit von Wasser aushärtet wird.

3. Verfahren gemäß Anspruch 2, **dadurch gekennzeichnet, dass** die silanterminierten Prepolymere (I) auf Polyisocyanaten oder Polyisocyanatgemischen mit ausschließlich aliphatisch und/oder cycloaliphatisch gebundenen Isocyanatgruppen und einer mittleren NCO-Funktionalität von 2 bis 4 basieren.

4. Verfahren gemäß Anspruch 2 oder 3, **dadurch gekennzeichnet, dass** als Additive (II) nichtionische Tenside auf Basis von Polyethersiloxanen eingesetzt werden.

5. Verfahren gemäß einem der Ansprüche 2 bis 4, **dadurch gekennzeichnet, dass** die aufzuschäumenden Zusammensetzungen bezogen auf Trockensubstanz 85 bis 99,9 Gewichtsteile silanterminierten Polyurethan-Prepolymers (I) und 0,1 bis 15 Gewichtsteile des (Schaum-) Additivs (II) sowie 0 bis 50 Gewichtsteile an Hilfs- und Zusatzstoffen (V) enthalten.

6. Verfahren gemäß einem der Ansprüche 2 bis 5, **dadurch gekennzeichnet, dass** zur Aushärtung Wasser zugesetzt wird, wobei die Menge so bemessen ist, dass das Molverhältnis von Alkoxysilangruppen des silanterminierten Prepolymers zu zugesetztem Wasser kleiner oder gleich 1 ist.

7. Wundauflagen erhältlich nach einem Verfahren gemäß einem der Ansprüche 2 bis 6.

8. Verwendung von Zusammensetzungen enthaltend
a) silanterminierte Polyurethan-Prepolymere (I) umfassend wenigstens eine Alkoxysilangruppe
b) (Schaum-)Additive (II)
c) gegebenenfalls Katalysatoren (III),
d) gegebenenfalls Treibmittel (IV) sowie
e) gegebenenfalls weitere Hilfs- und Zusatzstoffe (V)
für die Herstellung von Auflagen für die Wundbehandlung.
